# EUROPEAN PATENT APPLICATION

(11) **EP 4 652 934 A1**
(43) Date of publication of application: **26.11.2025**
(21) Application number: 24177469.4
(22) Date of filing: 22.05.2024
(51) Int. Cl.: A61B 6/50, G06T 7/00, G16H 30/40

(54) **COMPUTER-IMPLEMENTED METHOD FOR PROCESSING A MODEL OF A VESSEL STRUCTURE OF A PATIENT, PROCESSING DEVICE, COMPUTER PROGRAM AND ELECTRONICALLY READABLE STORAGE MEDIUM**

(71) Applicant: Siemens Healthineers AG, 91301 Forchheim (DE)
(72) Inventor: Dr. Wels, Michael, 96047 Bamberg (DE); Hopfgartner, Christian, 90763 Fürth (DE); Lades, Felix, 91052 Erlangen (DE); Dr. Sühling, Michael, 91052 Erlangen (DE)
(74) Representative: Siemens Healthineers Patent Attorneys

(57) **Abstract**

The invention relates to a computer-implemented method for processing a three-dimensional model of a vessel structure of a patient, the method comprising:
- providing the model of the vessel structure, the model comprising a topological graph representation of the vessel structure and first courses of centerlines (2, 5) of the vessels,
- receiving user input data describing at least one corrected course (7) of a chosen centerline (2),
- determining, from the user input data and the model,
- an affected section (9) of the chosen centerline (2) affected by the correction, and
- at least one non-affected section (10) of the chosen centerline (2) outside the affected section (9),
and, for each branch-off, in particular bifurcation (4), along the affected section (9)
- a modifiable section (11) of the respective branch-off centerline (5), wherein the modifiable section (11) is connected to the affected section (9), and
- a non-modifiable section (12) of the respective branch-off centerline (5) outside the affected section (9),

- determining a deformation field from the affected section (9), the non-affected section (10), the non-modifiable section (12), and the corrected course (7), such that the deformation field maps the first course of the chosen centerline (2) to the corrected course (7) of the chosen centerline (2) and does not change any non-modifiable sections (12),
- applying the deformation field to the affected section (9) and all modifiable sections (11) to determine updated courses to be stored in the model as new first courses.

## Description

The invention concerns a computer-implemented method for processing a model of a vessel structure of a patient, a processing device, a computer program and an electrically readable storage medium. Features and advantages described in connection with the method of the invention can also be implemented as corresponding features, units, or modules in the processing device and in the computer program, and vice versa.

In medical imaging, methods are known to acquire image data relating to vessel structures in a patient, for example blood vessel structures and in particular blood vessel trees, like the coronary artery tree. Three-dimensional patient images of vessel structures can be acquired, for example, using magnetic resonance imaging or computed tomography (CT), in particular computed tomography angiography (CTA). In particular, contrast agents may be used to provide improved contrast. Well-known algorithms, in particular analysis functions trained by machine learning, are used to derive geometrical, three-dimensional models of the vessel structure. In a common approach, centerlines of vessels may be automatically determined and/or vessel lumen segmentation may be performed. Exemplarily regarding centerline determination, it is referred to Y. Zheng et al., "Robust and Accurate Coronary Artery Centerline Extraction in CTA by Combining Model-Driven and Data-Driven Approaches", in: In International Conference on Medical Image Computing and Computer-Assisted Intervention, Nagoya, Japan, MICCAI '13, pages 74-81, Sept. 2013, or to Y. Zheng et al, "Model-driven centerline extraction for severely occluded major coronary arteries", in: Machine Learning in Medical Imaging, LNCS 7588, pages 10-18, 2012.

Automatically determined models may, however, still have inaccuracies. It was therefore proposed to provide users with the possibility to edit the model, in particular in a visualization of the model in the underlying three-dimensional patient image. Often, the views in which, for example, centerlines or boundaries may be edited by a user, are multiplanar reformations (MPRs) or curved planar reformations (CPRs). The CPRs may be defined to follow the course of the centerlines of a certain vessel. Such a method for correcting centerlines (and also vessel lumen segmentations) is, for example, described in M. Wels et al., "Intuitive and accurate patient-specific coronary tree modeling from cardiac computed-tomography angiography", in: Interactive Medical Image Computing (Int. Conf. Med. Image Comput. Comput.-Assist. Interv. 2016 Workshop Proceedings), Athens, GR, pages 86-93, Oct. 2016.

US 10,733,787 B2 discloses a method for interactively generating a geometric model of a vessel structure on the basis of three-dimensional image data of an examination region of interest of a patient. A representation of the vessel structure is determined on the basis of three-dimensional image data and a two-dimensional representation is determined on the basis of the determined representation using a preferably non-linear planar reformation of the three-dimensional vessel structure. Subsequently, boundary indicators which define the surface profile of the volume object are edited in the two-dimensional representation. Following the editing, a three-dimensional representation of the edited boundary indicators is generated by back-transforming the edited boundary indicators into three-dimensional space.

Regarding the three-dimensional model of the vessel structure, it has been proposed to model the vessel tree as a bundle of independent centerlines having common parts. In an improved approach, topological graphs, in particular trees, were used to describe the vessel structure in three-dimensional models. Such data structures are known from computer technology. In concrete embodiments, it was suggested to represent the entire geometric object as a proper computer-scientific tree having nodes with sequences of 3D points, which describe centerlines. Each node hence contains the course of the centerline of the respective vessel leading from the parent node to the respective current node, which can also be stored in other descriptions, for example as a function instead of a sequence of points. Each tree node further comprises pointers to its child nodes, which in turn describe the child vessel courses to these child nodes, allowing to model complex vascularization as a recursive hierarchical data structure. An editing and processing approach adapted for three-dimensional models using such topological graphs was described in a publication by M. Wels et al., "Consistent Hierarchical 3D Vessel Tree Editing", in: PLEASE ADD CITATION HERE! The publication describes seven use cases for editing of a model of a vessel structure.

However, this and related approaches suffer from drawbacks. Editing operations across bifurcations could change the tree topology in an undesired and clinically implausible manner. Parts of the previous vessel course tend to persist in the tree structure as additional vessels. This may even not be noticed by the user, as the superficial impression on the view, in which the editing is done, in particular a CPR view, which does not show the rest of the tree, suggests successful editing. Furthermore, non-connected vessel crossings may be introduced.

It is an object of the current invention to provide an improved processing approach based on user input data describing a course change of a centerline, which respects and preserves the underlying true physical topology of the vessel structure.

This object is achieved by providing a computer-implemented method, a processing device, a computer program and an electronically readable storage medium according to the independent claims. Advantageous embodiments are described by the dependent claims.

A method for processing a three-dimensional model of a vessel structure of a patient according to the invention comprises the steps of
- providing the model of the vessel structure, the model comprising a topological graph representation of the vessel structure and first courses of centerlines of the vessels,
- receiving user input data describing at least one corrected course of a chosen centerline,
- determining, from the user input data and the model,
   - an affected section of the chosen centerline affected by the correction, and
   - at least one non-affected section of the chosen centerline outside the affected section, and, for each branch-off, in particular bifurcation, along the affected section
   - a modifiable section of the respective branch-off centerline, wherein the modifiable section is connected to the affected section, and
   - a non-modifiable section of the respective branch-off centerline outside the affected section,
- determining a deformation field from the affected section, the non-affected section, the non-modifiable section, and the corrected course, such that the deformation field maps the first course of the chosen centerline to the corrected course of the chosen centerline and does not change any non-modifiable sections,
- applying the deformation field to the affected section and all modifiable sections to determine updated courses to be stored in the model as new first courses.

In an additional, optional step, the stored model with the new first courses can be outputted as a data set to a respective interface. The stored model may also be referred to as an "updated model". In this way, the stored model can be outputted, e.g. to one or more of a data connection, a data storage, data network, and a display device. Additionally or alternatively, a graphic representation of an updated three-dimensional model of the vessel structure, which is based on the stored model (or updated model), can be outputted, e.g. to one or more of a data connection, a data storage, data network, and a display device.

While this method may be applied to any vessel structure, which can be described as a (computer-scientific) topological graph, preferably, the vessel structure may be a blood vessel structure (vascular structure), in particular a coronary vessel tree. In particular regarding such a coronary vessel tree, but also in other applications (for example for blood vessel structures in the brain or other organs), the vessel structure may be described in the model as a tree, which will be often used as an illustrating example here. In other words, three-dimensional vessel trees can be represented in a model as (computer-scientific) trees as topological graphs. Information regarding the vessels (for example their boundaries and, in particular, the centerline courses), may be stored in edges and/or, preferably, in nodes. In preferred embodiments, in each node of the tree, the first course of the vessel segment leading from a previous node to the respective node and subsequent nodes may be stored.

Preferably, the model may be determined by evaluating a three-dimensional patient image of the vessel structure, in particular applying at least one trained evaluation function to the image data. In particular, the patient image may comprise a magnetic resonance image and/or a computed tomography image. Preferably, the patient image is an angiographic image. For example, an approach as described by Y. Zheng et al. in "Model-driven centerline extraction for severely occluded major coronary arteries", Machine Learning in Medical Imaging, LNCS 7588, Pages 10-18, 2012, or other known approaches may be used to extract centerlines and/or vessel boundaries/lumens from the patient image. The three-dimensional model may then be constructed from these determined vessel properties, but may, as already discussed, suffer from inaccuracies. Hence, the user is provided the option to correct the model, in particular regarding the courses of the centerlines.

For example, the user corrected course may be defined with respect to a planar reformatted image of a three-dimensional patient image showing the vessel structure, in particular a curved planar reformation (CPR) following the course of the chosen vessel (the vessel along the chosen centerline). Herein, the patient image is registered to the model, as will be the case anyways if the model is derived from the patient image. The user input data may be the result of an editing operation, in particular created by interaction with the depiction of one individual centerline - the chosen centerline - either on an MPR or a CPR view. Known techniques described in the above-mentioned publications by M. Wels et al. may also be applied here.

The invention provides a topology-preserving modification of the three-dimensional model based on user input data describing a corrected course of a chosen centerline. The steps of the method apply to continuous, that is non-interrupted, sections of the chosen centerline, while, of course, multiple such corrections may each be treated on their own, for example, successively. Hence, the affected section and any modifiable sections are understood to be continuous, i.e., not interrupted.

Preferably, the method described here is applied to corrections along the course of a centerline. That is, the steps of determining the deformation field as well as the step of applying the deformation field may be only performed if the affected section is located between non-affected sections. Other editing cases, for example corrected courses ending at a distance to an existing centerline, adding a new centerline to the vessel structure, or deleting a centerline shall not be treated using this new approach, since existing approaches already preserve the topology in such cases. For example, steps as described in "Consistent Hierarchical 3D Vessel Tree Editing" by M. Wels, as cited above, may be used.

It is noted that the present invention is particularly advantageous whenever branch-offs are affected, which is the non-trivial case in centerline correction, since it concerns multiple nodes and/or edges in the topological graph. Hence, preferably, the steps of determining the deformation field as well as the step of applying the deformation field may at least be performed if the affected section passes a branch-off, leading to the definition of a non-zero number of modifiable sections.

In the invention, it is proposed to determine a deformation field based on the corrected course in the affected section (known from the user input data), which can also be applied to the modifiable section, where the correction is not known from the user input data. To provide a proper definition of the deformation field, it does not change the course of the centerlines in the non-affected sections and the non-modifiable sections. In other words, the deformation field is hence determined not to modify the non-affected section and the non-modifiable section. The deformation field may then also be applied to the modifiable section, resulting in a smooth and consistent editing operation.

Advantageously, by only modifying the course in centerline sections which are already present in the topological graph, the overall graph topology is preserved during editing. Hence, the physical correlations describing the real-world vessel structure are upheld without producing any non-physical artifacts. The method described here is less prone to degeneration of the whole geometric vessel structure as editing and graph update are better encapsulated. The editing operation is not applied directly, but an intermediate deformation field is estimated, which is then applied to the graph. The steps described can, in particular, be implemented in real time, allowing smooth editing even of more complex vessel trees.

The edited/corrected three-dimensional model can be used in many applications. Geometrically modeling vessel trees is an important prerequisite when it comes to further analysis of vessel structures depicted in medical patient images, e.g., with regards to atherosclerosis or simulated fluid dynamics. Easy interactive modeling capabilities that help the user to model vessel geometry exactly to his needs is crucial for the acceptance of such analysis tools. The invention allows manual overriding of results generated by evaluation functions, in particular artificial intelligence (Al), while preserving the correct physical basis. Furthermore, interactive tools for acquiring ground-truth annotations for training and evaluating machine learning-based automatic evaluation solutions for vessel tracing and the like may also use the technique described here.

In concrete embodiments, the affected section is determined by
- running along the chosen centerline and determining, in particular orthogonal, distances between the first course and corrected course;
- defining the affected section as all parts of the centerline, where the distance is larger than an affection threshold.

Usually, the affection threshold may be very small, such that all editing operations of the user are taken into account. For example, the threshold may correspond to a smallest measurable distance, for example a voxel size.

Preferably, all modifiable sections are determined by calculating a distance measure to the affected section along the vessel structure, that is, first courses, and selecting parts having distance measure up to distance threshold. Hence, with knowledge about the affected part, other parts of the vessel tree may be assigned a distance measure to the affected section, i.e. the region where editing took place. Generally speaking, the distance threshold may chosen dependent on the size of the chosen vessel, in particular a diameter, and/or a strength of correction, in particular a maximum distance determined to define the affected section. For example, the distance threshold may be defined to comprise one to four times the diameter of the chosen vessel in the affected section and/or one to four times the maximum distance determined to define the affected section. It is important to emphasize that the distance measures are, of course, determined along the first courses, since non-connected vessel parts are, of course, less or even not affected by any correction (in the sense of a "pulling" or "pushing" of the chosen vessel). In concrete embodiments, a Dijkstra algorithm may be used to compute shortest distances in the topological graph.

In preferred embodiments, generally, the centerlines and the corrected course may be defined by representative centerline points, wherein the sections are defined as sets of centerline points. The distance of the points along the centerlines may, for example, follow a predefined definition scheme. The points may be uniformly distributed along the centerlines, in particular having the same distance along the centerlines, but may also be otherwise distributed, in particular according to curvature of the centerline, according to an underlying grid, and the like. If, as discussed above, in each node of the tree, the first course of the vessel segment leading from a previous node (parent node) to the respective node and subsequent nodes (child nodes) are stored, the points are stored in the nodes in this case. For example, in the case of a tree, each tree node may hold a set of centerline points, which can be understood as a 3D polygon and describes the first course of a respective vessel segment, with arbitrary and not necessarily uniform sampling. Each tree node further stores pointers to its child nodes, hence, for example, modeling vascularization as a hierarchical data structure.

Using points to describe the courses of centerlines has the advantage that only certain sampling positions have to be used to determine the deformation field, allowing robust and fast determination. Furthermore, regarding sets of points, in particular three-dimensional polygons, approaches for determining deformation fields have already been proposed in the state of the art for other applications. These approaches, accordingly modified, can also be applied in the present method.

When determining the deformation field and using points, the non-affected and non-modifiable sections can easily be modelled. Centerline points in the non-affected section and the non-modifiable section are simply considered stationary, modelling a constraint.

In concrete, advantageous embodiments, intermediate points on the affected section may be defined as positions of orthogonal distance from the centerline points of the corrected course to the affected section, wherein the three-dimensional deformation field is determined based on pairs of corrected course centerline points and respective intermediate points to describe the correction in the affected section. In other words, orthogonal projections of the intermediate points onto the corrected course yield the centerline points of the corrected course. In the non-affected section and in the non-modifiable section, further pairs may be defined by the stationary centerline points.

In any case, pairs of centerline points result, from which the deformation field can be easily estimated. In other words, the point pairs serve as samples for the continuous three-dimensional deformation field.

Here, in especially preferred embodiments, the deformation field may be interpolated using radial basis functions. From the point pairs and the radial basis functions, a linear system of equations is established, which may be solved using fast numerical methods, in particular solving functions. Preferably, least-squares solvers and/or single value decomposition (SVD) can be used. The point pairs can be reduced to a certain interpolation region around the affected section, such that stationary points which are further away can be excluded from the computation. This also prevents the introduction of numerical variations as these centerline points are supposed to remain at their original location anyways.

In an article by M. Botsch and L. Kobbelt, "Real-time shape editing using radial basis functions", in: Computer Graphics Forum, 24, 2005, pages 611-621, a related topology-preserving mesh surface editing strategy is proposed, the basic principles of which can also be applied in the currently described method. Mesh surface points are, however, less sensitive to where exactly on the surface they end up after editing, as this will not alter the surface itself. In the current invention, the expanded semantics of vessel centerline points, in particular in coronary tree structures, is additionally taken into account by applying constraints, such that the global influence of local editing operations on the overall shape can be restricted. These constraints comprise the centerline points in the non-affected and non-modifiable sections being stationary. In this manner, editing one chosen centerline cannot alter the course of a distant vessel.

Hence, generally speaking, the deformation field may be determined by interpolation using radial basis functions. Once the deformation field has been determined, it is applied to the affected section and the modifiable sections, in particular their centerline points. This may lead to deviations from a point distribution scheme, if used.

Hence, in preferred embodiments, at least for the affected section and all modifiable sections, the centerline points of the updated courses may be adapted according to a point distribution scheme, in particular uniform point distribution. In this optional step, the resulting centerline points can be resampled to match the original sampling convention, that is, point distribution scheme, of the topological graph. In particular, a uniform point distribution may be required for analysis and/or postprocessing functions to be applied to the edited model of the vessel structure. However, in preferred embodiments, the adaptation may alternatively or additionally comprise deleting at least one centerline point where a centerline point density along the updated course is higher than a predetermined density defined by the point distribution scheme. In this manner, "squeezed" distributions, in particular in modifiable sections, may be resolved.

The invention also concerns a processing device, comprising at least one processor and at least one storage means, wherein the processing device further comprises, for processing a three-dimensional model of a vessel structure of a patient:
- a first interface for receiving the model of the vessel structure, the model comprising a topological graph representation of the vessel structure and first courses of centerlines of the vessels,
- a second interface for receiving user input data describing at least one corrected course of a chosen centerline,
- a first determination unit for determining, from the user input data and the model,
   - an affected section of the chosen centerline affected by the correction, and
   - at least one non-affected section of the chosen centerline outside the affected section, and, for each branch-off, in particular bifurcation, along the affected section,
   - a modifiable section of the respective branch-off centerline, wherein the modifiable section is connected to the affected section, and
   - a non-modifiable section of the respective branch-off centerline outside the affected section,
- a second determination unit for determining a deformation field from the affected section, the non-affected section, the non-modifiable section, and the corrected course, such that the deformation field maps the first course of the chosen centerline to the corrected course of the chosen centerline and does not change any non-modifiable sections,
- an updating unit for applying the deformation field to the affected section and all modifiable sections to determine updated courses to be stored in the model as new first courses.

The processing device may further comprise a third interface for outputting the updated model. In this way, the stored or updated model can be outputted, e.g. to one or more of a data connection, a data storage, data network, and a display device. Additionally, or alternatively, a graphic representation of an updated three-dimensional model of the vessel structure, which is based on the stored model (or updated model), can be outputted, e.g. to one or more of a data connection, a data storage, data network, and a display device.

All features and remarks regarding the method according to the invention may also be applied to the processing device according to the invention and vice versa, such that the same advantages can be achieved.

In particular, the processing device may be part of a control device of a medical imaging device, for example an x-ray device and/or a magnetic resonance device, and/or a workstation. In particular, at least one input device and at least one output device may be associated to the processing device. Using the output device, visualizations of the model, in particular the mentioned MPRs and/or CPRs, may be output, comprising respective image data as a basis for assessment by a user. User input data may be generated by the input device, in particular when the user interacts with the visualization to perform an editing operation on the chosen centerline.

A computer program according to the invention comprises program means such that, when the computer program is executed on a processing device, the processing device is caused to perform the steps of a method according to the invention. The computer program may be stored on an electronically readable storage medium according to the invention, which thus has control information stored thereon, the control information comprising at least one computer program according to the invention, such that, when the storage medium is used in a processing device, the processing device is configured to perform a method according to the invention. The storage medium according to the invention may be non-transient, for example a CD-ROM.

Other objects and features of the present invention will become apparent from the following detailed description considered in conjunction with the accompanying drawings. The drawings, however, are only principal sketches designed solely for the purpose of illustration and do not limit the invention. The drawings show:
- Fig. 1: a flowchart of an embodiment of a method according to the invention,
- Fig. 2: a first schematical illustration showing a tree segment of a topological tree structure of a vessel structure and a user-defined corrected course of a chosen centerline,
- Fig. 3: a second schematical illustration showing distances from an affected section and corresponding definitions,
- Fig. 4: a third schematical illustration showing the definition of centerline point pairs as samples for determining a deformation field,
- Fig. 5: a fourth schematical illustration showing the tree segment deformed by the deformation field,
- Fig. 6: a fifth schematical illustration showing resampled centerline points, and
- Fig. 7: the functional structure of a processing device according to the invention.

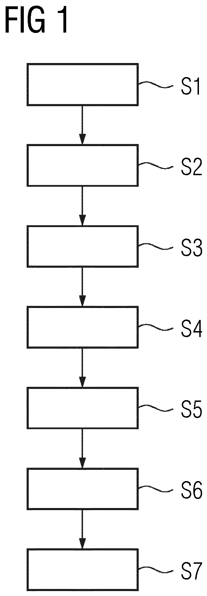

Fig. 1 is a flowchart of an embodiment of a method according to the invention for processing a three-dimensional model of a vessel structure of a patient, for example a coronary artery tree. The method may be embedded into an editing module, where a user can perform editing operations on at least the centerlines of the model. The method described here handles editing operations that change the first course of a chosen centerline to a corrected course in an affected section between two non-affected sections of the chosen centerline and is at least applied if there is at least one bifurcation in the affected section.

In a step S1, the three-dimensional model is received via a first interface. In a step S2, user input data describing the corrected course is received as result of an editing operation via a second interface. The model has been determined by evaluating a three-dimensional patient image of the vessel structure using a trained evaluation function. The patient image may comprise a magnetic resonance image and/or a computed tomography image. Centerlines and/or vessel boundaries/lumens have been extracted from the patient image and the three-dimensional model was constructed from these determined vessel properties. Here, since a vessel tree is concerned as the vessel structure, the model comprises a tree representation of the vessel structure. The tree is an example of a topological graph. In this embodiment, each node of the tree stores the first course of the vessel segment leading from a previous node (parent node) to the respective node and subsequent nodes (child nodes). The first courses are stored as sets of three-dimensional centerline points, which may follow a certain point distribution scheme. In the embodiment presented here, uniform point distribution is used, but other schemes may also be employed.

Since the automatically determined model may suffer from inaccuracies, the user is provided the option to correct the model, in particular regarding the courses of the centerlines. Here, the corrected course may be defined by interacting with a planar reformatted image of the three-dimensional patient image, in particular a curved planar reformation (CPR) following the course of the chosen vessel.

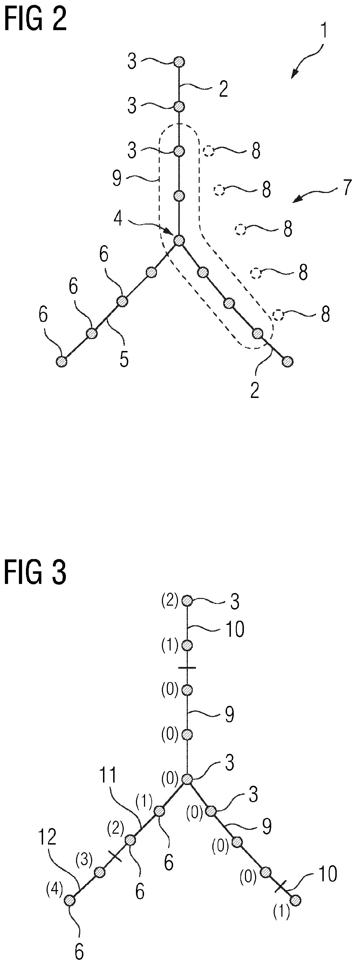

Fig. 2 schematically shows a tree segment 1 of the modelled vessel structure. It is noted that the simplified view is provided for better explanation of the applied processing method. In reality, vessel structures are more complex regarding size and degree of ramification.

As can be seen, the chosen centerline 2 is defined by multiple centerline points 3. At a bifurcation 4, a branch-off centerline 5 with respective centerline points 6 leads away from the chosen centerline 2. Hence, a node is defined at the bifurcation 4. Fig. 2 also shows the user corrected course 7, which also has centerline points 8. The corrected course 7 spans the bifurcation 4.

Returning to Fig. 1, in a step S3, certain sections are determined using the model and the user input data. First of all, an affected section 9 already indicated in Fig. 1 is determined as the section of the chosen centerline 2 where the centerpoints 8 of the corrected course 7 deviate from the centerline points 3 of the first course of the chosen centerline 2, in particular by more than an affection threshold. All other centerline points 3 of the chosen centerline 2 belong to non-affected sections 10 (see Fig. 3).

For all branch-off centerlines 5, distance measures along the first courses in the vessel structure to the affected sections 9 are now determined using a Dijkstra algorithm. In the example presented here, due to the uniform distribution, the distance measures can be expressed exemplarily as integers, shown in Fig. 3 in parentheses. All centerline points 6 along the branch-off centerline 5, whose distance measure is smaller than a distance threshold, in this case three, are added to a modifiable section 11, which, consequently, is connected to the affected section, as indicated in Fig. 3. All other centerline points 6 of the branch-off centerline 5 belong to a non-modifiable section 12.

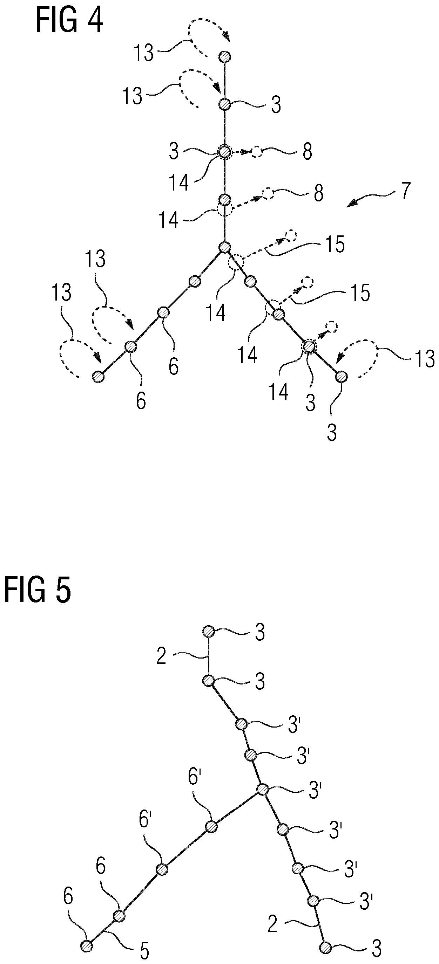

In a step S4 of Fig. 1, a deformation field is then determined. In a first substep, centerline point pairs are defined, which serve as a basis for interpolating the deformation field. As the centerline points 3 and 6 of the non-affected and non-modifiable sections 10, 12, respectively, are supposed to be stationary, as indicated by arrows 13 in Fig. 4, centerline point pairs can easily be generated with themselves. These pairs serve as constraints. In the affected section 9, however, intermediate points 14 (dotted) are defined as positions of orthogonal distance from the centerline points 8 of the corrected course 7 to the affected section 9. This can be understood as a resampling of the affected section 9 to match the sampling of the edited, corrected course 7. Pairs of corrected course centerline points 8 and respective intermediate points 14 are formed to describe the correction in the affected section 9 (indicated by arrows 15) and serve as samples for the three-dimensional deformation field.

In a second substep of step S4, the deformation field is determined by interpolation using radial basis functions. A system of linear equations is defined and solved using a solving function, for example employing SVD.

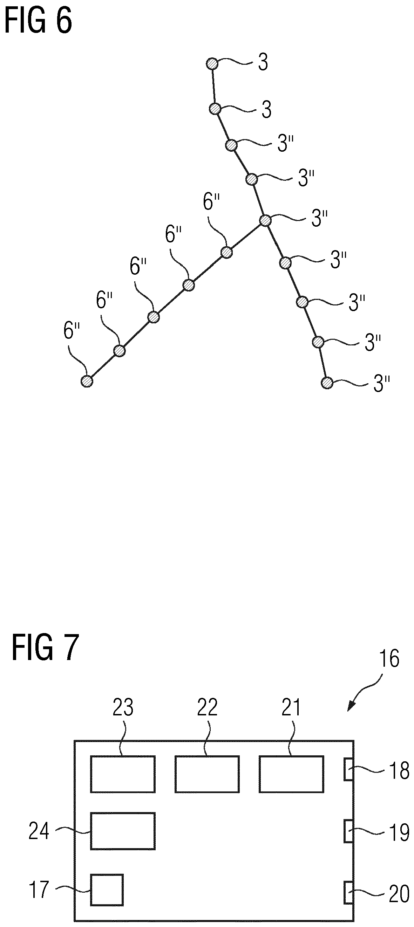

In a step S5, the determined deformation field is then applied to the affected section 9 and the modifiable section 11, resulting in new centerline points 3' and 6', respectively. As can be seen, these centerline points 3' and 6' are not according to the uniform point distribution scheme, such that, in an optional step S6, they may be adapted to uniformly distributed centerline points 3" and 6" as shown in Fig. 6. The step S6 may also, independently from the uniform point distribution scheme, serve to delete centerline points 3', 6' if they are to close due to "squeezing" by the deformation field.

In a step S7, the updated courses are stored as new first courses in the model and the updated model is output via a third interface.

Additionally in step S7, the stored or updated model can be outputted, e.g. to one or more of a data connection, a data storage, data network, and a display device. Additionally, or alternatively, a graphic representation of an updated three-dimensional model of the vessel structure, which is based on the stored model (or updated model), can be outputted, e.g. to one or more of a data connection, a data storage, data network, and a display device.

Fig. 7 shows a functional drawing of a processing device 16. The processing device, which may be part of a control device of a medical imaging device or of a workstation, comprises a storage means 17, a first interface 18 for receiving the model in step S1, a second interface 19 for receiving the user input data in step S2 and a third interface 20 for outputting the updated model in step S7. In a first determination unit 21, the affected section 9, the non-affected sections 10, the modifiable sections 11 and the non-modifiable sections 12 are determined according to step S3. The processing device further comprises a second determination unit 22 for determining the deformation field according to step S4. In an updating unit 23, the deformation field is applied to the model according to step S5. In an optional adaptation unit 24, the centerline point distribution may be adapted according to step S6.

Independent of the grammatical term usage, individuals with male, female or other gender identities are included within the term "patient".

## Claims

1. Computer-implemented method for processing a three-dimensional model of a vessel structure of a patient, the method comprising:
- providing the model of the vessel structure, the model comprising a topological graph representation of the vessel structure and first courses of centerlines (2, 5) of the vessels,
- receiving user input data describing at least one corrected course (7) of a chosen centerline (2),
- determining, from the user input data and the model,
- an affected section (9) of the chosen centerline (2) affected by the correction, and
- at least one non-affected section (10) of the chosen centerline (2) outside the affected section (9),
and, for each branch-off, in particular bifurcation (4), along the affected section (9)
- a modifiable section (11) of the respective branch-off centerline (5), wherein the modifiable section (11) is connected to the affected section (9), and
- a non-modifiable section (12) of the respective branch-off centerline (5) outside the affected section (9),
- determining a deformation field from the affected section (9), the non-affected section (10), the non-modifiable section (12), and the corrected course (7), such that the deformation field maps the first course of the chosen centerline (2) to the corrected course (7) of the chosen centerline (2) and does not change any non-modifiable sections (12),
- applying the deformation field to the affected section (9) and all modifiable sections (11) to determine updated courses to be stored in the model as new first courses.

2. Method according to claim 1, **characterized in that** the affected section (9) is determined by
- running along the chosen centerline (2) and determining, in particular orthogonal, distances between the first course and the corrected course (7);
- defining the affected section (9) as all parts of the chosen centerline (2), where the distance is larger than an affection threshold.

3. Method according to claim 1 or 2, **characterized in that** all modifiable sections (11) are determined by calculating a distance measure to the affected section (9) along the vessel structure and selecting parts having distance measure up to distance threshold.

4. Method according to claim 3, **characterized in that** the distance threshold is chosen dependent on the size of the vessel of the chosen centerline (2), in particular a diameter, and/or a strength of correction, in particular a maximum distance determined to define the affected section (9).

5. Method according to one of the preceding claims, **characterized in that** the centerlines (2, 5) and the corrected course (7) are defined by representative centerline points (3, 6, 8), wherein the sections (9, 10, 11, 12) are defined as sets of centerline points (3, 6).

6. Method according to claim 5, **characterized in that** intermediate points (14) on the affected section (9) are defined as positions of orthogonal distance from the centerline points (8) of the corrected course (7) to the affected section (9), wherein the three-dimensional deformation field is determined based on pairs of corrected course centerline points (8) and respective intermediate points (14) to describe the correction in the affected section (9).

7. Method according to claim 5 or 6, **characterized in that**, at least for the affected section (9) and all modifiable sections (11), the centerline points (3, 3', 6, 6') of the updated courses are adapted according to a point distribution scheme, in particular uniform point distribution.

8. Method according to claim 7, **characterized in that** the adaptation comprises deleting at least one centerline point (3') where a centerline point density along the updated course is higher than a predetermined density defined by the point distribution scheme.

9. Method according to one of the preceding claims, **characterized in that** the deformation field is determined by interpolation using radial basis functions.

10. Method according to one of the preceding claims, **characterized in that** the vessel structure is described in the model as a tree, wherein, in particular, in each node of the tree, the first course of the vessel segment leading from a previous node to the respective node and subsequent nodes are stored.

11. Method according to one of the preceding claims, **characterized in that** the steps of determining the deformation field as well as the step of applying the deformation field are only performed if the affected section (9) passes a branch-off and/or if the affected section is located between non-affected sections (10).

12. Method according to one of the preceding claims, **characterized in that** the model is determined by evaluating a three-dimensional patient image of the vessel structure, in particular applying at least one trained evaluation function to the image data.

13. Processing device (16), comprising at least one processor and at least one storage means (17), wherein the processing device (16) further comprises, for processing a three-dimensional model of a vessel structure of a patient:
- a first interface (18) for receiving the model of the vessel structure, the model comprising a topological graph representation of the vessel structure and first courses of centerlines (2, 5) of the vessels,
- a second interface for receiving user input data describing at least one corrected course (7) of a chosen centerline (2),
- a first determination unit (21) for determining, from the user input data and the model,
- an affected section (9) of the chosen centerline (2) affected by the correction, and
- at least one non-affected section (10) of the chosen centerline (2) outside the affected section (9),
and, for each branch-off, in particular bifurcation (4), along the affected section (9)
- a modifiable section (11) of the respective branch-off centerline (5), wherein the modifiable section (11) is connected to the affected section (9), and
- a non-modifiable section (12) of the respective branch-off centerline (5) outside the affected section (9),
- a second determination unit (22) for determining a deformation field from the affected section (9), the non-affected section (10), the non-modifiable section (11), and the corrected course (7), such that the deformation field maps the first course of the chosen centerline (2) to the corrected course (7) of the chosen centerline (2) and does not change any non-modifiable sections (11),
- an updating unit (23) for applying the deformation field to the affected section (9) and all modifiable sections (11) to determine updated courses to be stored in the model as new first courses:

14. Computer program, which, when it is executed on a processing device (16), causes the processing device (16) to perform the steps of a method according to one of the claims 1 to 12.

15. Electronically readable storage medium, on which a computer program according to claim 14 is stored.
